(19) Europäisches Patentamt
European Patent Office
Office européen des brevets

(11) **EP 3 815 788 A1**

(12) **EUROPÄISCHE PATENTANMELDUNG**

(43) Veröffentlichungstag:
**05.05.2021 Patentblatt 2021/18**

(21) Anmeldenummer: **20204424.4**

(22) Anmeldetag: **28.10.2020**

(51) Int Cl.:
**B01L 3/02** (2006.01)        **B01L 3/00** (2006.01)
**C12M 1/32** (2006.01)        **C12M 1/12** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **31.10.2019 DE 102019216886**

(71) Anmelder:
• **Albert-Ludwigs-Universität Freiburg**
**79098 Freiburg (DE)**
• **Hahn-Schickard-Gesellschaft für angewandte Forschung e.V.**
**78052 Villingen-Schwenningen (DE)**

(72) Erfinder:
• **Tröndle, Kevin**
**79104 Freiburg (DE)**
• **Peter, Koltay**
**79117 Freiburg (DE)**
• **Sabrina, Kartmann**
**78052 Villingen-Schwenningen (DE)**
• **Jeney, Csaba**
**78052 Villingen-Schwenningen (DE)**

(74) Vertreter: **Stöckeler, Ferdinand**
**Schoppe, Zimmermann, Stöckeler**
**Zinkler, Schenk & Partner mbB**
**Patentanwälte**
**Radlkoferstrasse 2**
**81373 München (DE)**

(54) **TRANSFER ZUMINDEST EINES PARTIKELS IN EINER FLÜSSIGKEIT VON EINER ABGABEEINHEIT ZU EINER EMPFANGSEINHEIT**

(57) Bei einem Verfahren zum Transferieren zumindest eines Partikels von einer Abgabeeinheit zu einer Empfangseinheit wird ein Flüssigkeitstropfens, in dem der zumindest eine Partikel angeordnet ist, an einer Abgabeposition der Abgabeeinheit bereitgestellt, wobei der Flüssigkeitstropfen aufgrund von Oberflächenkräften an der Abgabeposition gehalten wird. Die Abgabeposition der Abgabeeinheit ist einer Empfangsposition der Empfangseinheit zugewandt ist. Die Abgabeeinheit und die Empfangseinheit werden synchron gedreht, um den Partikel und zumindest einen Teil des Flüssigkeitstropfens, in dem Partikel angeordnet ist, durch Zentrifugalkraft von der Abgabeposition der Abgabeeinheit zu der Empfangsposition der Empfangseinheit zu transferieren.

## Beschreibung

### Technisches Gebiet

Die vorliegende Erfindung bezieht sich auf Verfahren zum Transferieren zumindest eines Partikels in einer Flüssigkeit von einer Abgabeeinheit zu einer Empfangseinheit. Insbesondere beziehen sich Beispiele der vorliegenden Erfindung auf solche Verfahren, die in partikelbasierten Mikroanalysesystemen Anwendung finden können, bei denen bestimmte Prozessierungsprotokolle einen Transfer von in einer Flüssigkeit befindlichen Partikeln von einer ersten Oberfläche zu einer zweiten Oberfläche beinhalten.

### Hintergrund

Aus der WO 2015/158777 A1 ist ein Verfahren bekannt, bei dem Zellen auf eine beliebige Oberfläche aufgebracht werden, wobei die Fähigkeit der Zellen, an der Oberfläche zu haften, überprüft wird. Nicht anhaftende Zellen werden verworfen und die anhaftenden Zellen werden abgelöst und in einen 3D-Gewebe-Kultivierungsprozess transferiert.

Aus der DE 10 2007 018056 A1 sind Verfahren zur Tropfenmanipulation bekannt, bei denen mindestens ein Tropfen in Kontakt mit einer festen Haltefläche einer Tropfenhalterung bereitgestellt wird. Ein Abtropfen kann durch verschieden Mechanismen erreicht werden. Die beschriebenen Methoden umfassen Kräfte, welche durch ein elektrisches Feld erzeugt werden, so dass eine Kraft auf den mindestens einen Tropfen ausgeübt wird. Ferner kann ein Abtropfen durch eine Durchmesservergrößerung erfolgen, wobei mehrere Tropfen kombiniert werden und der Abtropfvorgang durch Gravitation erfolgt. Ferner ist eine Abtropfeinrichtung beschrieben, bei der der Tropfen über eine Kante bewegt wird.

Derzeit werden, um Partikel, insbesondere zellbasierte Partikel, zu transferieren, ausschließlich spezialisierte Plattformen verwendet. Solche mikrostrukturierten spezialisierten Plattformen sind jedoch nachteilig dahingehend, dass sie kostenaufwändig sind, dass die Anordnung der Partikel/Tropfen auf das vorgegebene Design limitiert ist, dass der Partikeltransfer manuell erfolgt, und dass nach dem Transfer kein Rück-Transfer möglich ist.

### Überblick

Es besteht ein Bedarf nach Verfahren, die einen Transfer eines oder mehrerer Partikel, und insbesondere eines oder mehrerer Partikel, die jeweils in einem Flüssigkeitstropfen angeordnet sind, ermöglichen.

Die vorliegende Erfindung schafft ein Verfahren nach Anspruch 1.

Ein Beispiel der Erfindung schafft ein Verfahren zum Transferieren zumindest eines Partikels in einer Flüssigkeit von einer Abgabeeinheit zu einer Empfangseinheit, mit folgenden Merkmalen:

Bereitstellen eines Flüssigkeitstropfens, in dem der zumindest eine Partikel angeordnet ist, an einer Abgabeposition der Abgabeeinheit, wobei der Flüssigkeitstropfen aufgrund von Oberflächenkräften an der Abgabeposition gehalten wird, wobei die Abgabeposition der Abgabeeinheit einer Empfangsposition der Empfangseinheit zugewandt ist; und

synchrones Drehen der Abgabeeinheit und der Empfangseinheit, um den Partikel und zumindest einen Teil des Flüssigkeitstropfens, in dem Partikel angeordnet ist, durch Zentrifugalkraft von der Abgabeposition der Abgabeeinheit zu der Empfangsposition der Empfangseinheit zu transferieren.

Erfindungsgemäß wird der Transfer eines oder mehrerer Partikel, die sich jeweils in einem Flüssigkeitstropfen befinden bzw. in demselben suspensiert sind, von einer Abgabeeinheit zu einer Empfangseinheit zentrifugal induziert, so dass es möglich ist, einen oder gleichzeitig mehrere Partikel parallel zu übertragen. Dazu werden die Abgabeeinheit und die Empfangseinheit synchron miteinander gedreht, so dass jeweilige Abgabepositionen und Empfangspositionen ausgerichtet zueinander bleiben. Somit können gleichzeitig mehrere Partikel entsprechend von definierten Abgabepositionen zu definierten Empfangspositionen transferiert werden.

Bei Beispielen ist die Abgabeposition der Abgabeeinheit eine flache Oberfläche. Der oder die Flüssigkeitstropfen werden auf der flachen Oberfläche lediglich durch die wirkenden Oberflächenkräfte gehalten, so dass strukturierte Fluidikstrukturen zum Halten des oder der Flüssigkeitstropfen vor dem Transfer derselben nicht notwendig sind. Die Oberflächenkräfte (Grenzflächenenergie) beinhalten dabei neben der Oberflächenspannung der Flüssigkeit, also der Flüssigkeit-Gas-Wechselwirkung zwischen der Flüssigkeit und der Umgebung, wie z.B. Luft, auch Flüssigkeit-Festkörper-Wechselwirkungen zwischen der Flüssigkeit und der Abgabeeinheit.

Bei Beispielen weist die Abgabeeinheit eine flache Oberfläche auf, wobei das Bereitstellen des Flüssigkeitstropfens ein Bereitstellen einer Mehrzahl von Flüssigkeitstropfen, in denen jeweils zumindest ein Partikel angeordnet ist, in einem zweidimensionalen Muster an jeweiligen Abgabepositionen auf der flachen Oberfläche aufweist. Somit ist es möglich, mehrere Partikel parallel zu einer Empfangseinheit zu transferieren, indem die Abgabeeinheit mit einer Rotation beaufschlagt wird. Beim Drehen der Abgabeeinheit und der Empfangseinheit können dann die in den jeweiligen Flüssigkeitstropfen angeordneten Partikel zu mit den Abgabepositionen ausgerichteten Empfangspositionen der Empfangseinheit transferiert werden.

Bei Beispielen ist während des Drehens ein Abstand zwischen der Abgabeposition und der Empfangsposition oder zwischen den Abgabepositionen und den Empfangspositionen konstant. Bei Beispielen wird die Abgabeeinheit parallel zu der Empfangseinheit angeordnet und dieselben bilden eine Verbundanordnung, wobei die Positionsbeziehung zwischen Abgabeeinheit und Empfangseinheit während des Drehens nicht geändert wird. Bei Beispielen kann ein geringer Neigungswinkel zwischen den sich gegenüberliegenden Oberflächen der Abgabeeinheit und der Empfangseinheit vorgesehen sein, der während des Drehens konstant ist.

Bei Beispielen kann das zweidimensionale Muster, in dem die Flüssigkeitstropfen auf der Abgabeeinheit erzeugt werden, der Anordnung von Kavitäten in einer Mikrotiterplatte entsprechen. Bei solchen Beispielen kann die Empfangseinheit eine Mikrotiterplatte sein. Die Abgabeeinheit und die Mikrotiterplatte können vor dem Drehen derart zueinander angeordnet werden, dass jeweils zumindest einer der Flüssigkeitstropfen auf der Abgabeeinheit mit einer Kavität in der Mikrotiterplatte ausgerichtet ist. Indem die Verbundanordnung aus Abgabeeinheit und Mikrotiterplatte dann miteinander gedreht werden, können somit der oder die mit einer jeweiligen Kavität der Mikrotiterplatte ausgerichtete oder ausgerichteten Partikel in die jeweilige Kavität transferiert werden. Bei Beispielen kann die Abgabeeinheit somit ein Deckel für die Mikrotiterplatte sein, der an der Mikrotiterplatte derart angebracht wird, dass die jeweiligen Kavitäten der Mikrotiterplatte und die am Deckel anhaftenden Flüssigkeitstropfen derart angeordnet werden, dass mindestens ein Flüssigkeitstropfen über jeweils einer der Kavitäten angeordnet ist. Bei Beispielen können die Kavitäten der Mikrotiterplatte durch den Deckel geschlossen werden und jeweils zumindest einer der Flüssigkeitstropfen in jeweils einer der Kavitäten angeordnet werden.

Bei Beispielen kann das Verfahren ein Einbringen der Abgabeeinheit und der Empfangseinheit, wie z. B. der Mikrotiterplatte mit dem darauf angeordneten Deckel, in eine Zentrifuge aufweisen. Bei Beispielen kann die Mikrotiterplatte in einer waagrechten Stellung in eine Schwenkvorrichtung einer Zentrifuge eingebracht werden. Die Schwenkvorrichtung der Mikrotiterplatte kann ausgebildet sein, um die Mikrotiterplatte dadurch, dass die Zentrifuge sich dreht, aus einer Ausgangsstellung in eine Abgabestellung zu schwenken. In der Abgabestellung können die Abgabepositionen radial innerhalb der Empfangspositionen sein, so dass die Partikel und zumindest Teile der Flüssigkeitstropfen, in denen die Partikel angeordnet sind, von den Abgabepositionen zu den Empfangspositionen transferiert werden können.

Bei Beispielen kann oder können der oder die Flüssigkeitstropfen, in denen jeweils zumindest ein Partikel angeordnet ist, unter Verwendung einer Dispenservorrichtung auf die Abgabeeinheit aufgebracht werden. Bei Beispielen kann hierzu eine automatische Positionierungseinrichtung verwendet werden, die ausgelegt sein kann, um die Dispenservorrichtung und/oder die Abgabeeinheit zu bewegen, um so ein automatisches Ändern der Positionsbeziehung zwischen Dispenservorrichtung und Abgabeeinheit zu bewirken. Somit ist es möglich, eine Mehrzahl der Flüssigkeitstropfen, in denen jeweils zumindest ein Partikel angeordnet ist, in einem zweidimensionalen Muster auf der Abgabeeinheit zu erzeugen. Ferner kann eine Positionierungseinrichtung vorgesehen sein, die ausgelegt ist, um die Abgabeeinheit relativ zu der Empfangseinheit zu positionieren und/oder die Verbundanordnung aus Abgabeeinheit und Empfangseinheit in einer Zentrifuge zu positionieren. Dadurch kann das Verfahren automatisiert werden, wobei entsprechend Einrichtungen beispielsweise durch Roboter implementiert sein können. Bei Beispielen kann die Dispensiervorrichtung als Druckkopf implementiert sein, der beispielsweise durch eine Positionierungseinrichtung in zwei oder drei Richtungen eines dreidimensionalen Koordinatensystems beweglich ist, um ihn relativ zu der Abgabeeinheit zu positionieren.

Bei Beispielen liegt das Volumen des oder der Flüssigkeitstropfen in einem Bereich von 0,5 $\mu$l bis 100 $\mu$l. Bei Beispielen kann der Kontaktwinkel zwischen der Flüssigkeit des Flüssigkeitstropfens oder der Flüssigkeitstropfen und dem Material der Abgabeposition oder den Abgabepositionen der Abgabeeinheit 45° bis 135° betragen. Dadurch können der oder die Flüssigkeitstropfen lediglich durch Oberflächenkräfte an der oder den Abgabepositionen gehalten werden, die stärker sind als die Erdgravitationskraft, die auf den oder die Flüssigkeitstropfen wirkt. Bei Beispielen können der oder die Partikel ein solides Material, ein halbfestes Material, zellbasierte Partikel aus biologischen Zellen, und/oder Kombinationen dieser Bestandteile aufweisen. Beispiele eignen sich somit insbesondere zur Anwendung in Mikroanalysesystemen, bei denen zellbasierte Partikel aus biologischen Zellen zwischen Oberflächen transferiert werden sollen.

Beispiele der vorliegenden Erfindung werden nachfolgend bezugnehmend auf die beigefügten Zeichnungen beschrieben. Es zeigen:

Fig. 1     eine schematische Darstellung zur Erläuterung von Beispielen der Erfindung zugrundeliegenden Konzepts;

Fig. 2     ein Flussdiagramm eines Beispiels eines erfindungsgemäßen Verfahrens;

Fig. 3     ein Flussdiagramm eines weiteren Beispiels eines erfindungsgemäßen Verfahrens;

Fig. 4     eine schematische Draufsicht auf ein Beispiel einer Mikrotiterplatte;

Fig. 5     eine schematische Draufsicht auf ein Beispiel einer Abgabeeinheit; und

Fig. 6     eine schematische Darstellung eines Beispiels einer Zentrifuge mit einer Verbundanordnung aus Abgabeeinheit und Empfangseinheit.

Im Folgenden werden Beispiele der vorliegenden Erfindung detailliert und unter Verwendung der beigefügten Zeichnungen beschrieben. Es sei darauf hingewiesen, dass gleiche Elemente oder Elemente, die die gleiche Funktionalität aufweisen, mit gleichen oder ähnlichen Bezugszeichen versehen sein können, wobei eine wiederholte Beschreibung von Elementen, die mit dem gleichen oder ähnlichem Bezugszeichen versehen sind, typischerweise weggelassen wird. Beschreibungen von Elementen, die gleiche oder ähnliche Bezugszeichen aufweisen, sind gegeneinander austauschbar. In der folgenden Beschreibung werden viele Details beschrieben, um eine gründlichere Erklärung von Beispielen der Erfindung zu liefern. Es ist jedoch für Fachleute offensichtlich, dass andere Beispiele ohne diese spezifischen Details implementiert werden können. Merkmale der unterschiedlichen beschriebenen Beispiele können miteinander kombiniert werden, es sei denn, Merkmale einer entsprechenden Kombination schließen sich gegenseitig aus oder eine solche Kombination ist ausdrücklich ausgeschlossen.

Beispiele der vorliegenden Erfindung beziehen sich auf Verfahren zum Transfer von Partikeln, die in mindestens einem Flüssigkeitstropfen suspensiert sind, der auf einer ersten Oberfläche, beispielsweise einem Substrat, sitzt, auf eine zweite Oberfläche, die als Rezipient dient. Dort stehen der oder die Partikel dann zu einer weiteren Analyse oder Verwendung zur Verfügung. Ziel des Transfers kann es bei Beispielen der Erfindung dabei sein, Partikel in getrennte Reaktionskammern zu überführen, d.h. zu separieren, Partikel zur Weiterverarbeitung in ein gemeinsames Reservoir zu überführen, d.h. zu sammeln, oder eine Trägerflüssigkeit des oder der Partikel zu tauschen, d.h. zu waschen oder zu behandeln.

Beispiele der vorliegenden Erfindung beziehen sich auf Verfahren zum Transfer zellbasierter Partikel, wobei diese Partikel in Flüssigkeitstropfen auf einem Substrat erzeugt und anschließend durch Zentrifugation in eine Mikrotiterplatte, beispielsweise eine 384-Kammer-Mikrotiterplatte transferiert werden können. Es ist jedoch klar, dass bei anderen Beispielen andere Mikrotiterplatten mit einer unterschiedlichen Anzahl von Kavitäten verwendet werden können. Bei Beispielen kann sich im Endzustand genau ein Partikel in jeder Kammer der Mikrotiterplatte befinden.

Beispiele der vorliegenden Erfindung ermöglichen den Transfer von mindestens einem Partikel in einer Flüssigkeit (Probe), beispielsweise in Tropfenform, von einer Oberfläche (Substrat) zu einer in radialer Richtung gegenüberliegenden Oberfläche (Rezipient) durch die Einwirkung einer gerichteten Kraft auf Flüssigkeit und Partikel. Erfindungsgemäß ist die Kraft die Zentrifugalkraft und wird durch eine Drehung der sich gegenüberliegenden Oberflächen erzeugt.

Zur Probenvorbereitung können dabei Partikel direkt auf einem Substrat (Abgabeeinheit) abgelegt werden. Dabei können zellbasierte Partikel, wie z.B. Sphäroide, direkt erzeugt werden, beispielsweise unter Verwendung einer sogenannten Hanging-Drop-Methode. Die Partikel werden dann, beispielsweise zur weiteren Analyse, auf den Rezipienten (die Empfangseinheit) übertragen. Hierzu wird durch Zentrifugation eine Kraft erzeugt, welche von der Abgabeeinheit, d.h. der Tropfenoberfläche, zum Rezipienten gerichtet ist. Bei ausreichend großer Zentrifugalkraft wird der Partikel inklusive eines Teils der umgebenden Flüssigkeit als frei fliegender Tropfen, vom Substrat abgelöst und auf den Rezipienten übertragen. Die weitere Verarbeitung bzw. Analyse der Probe/des Partikels, kann dann auf dem Rezipienten erfolgen.

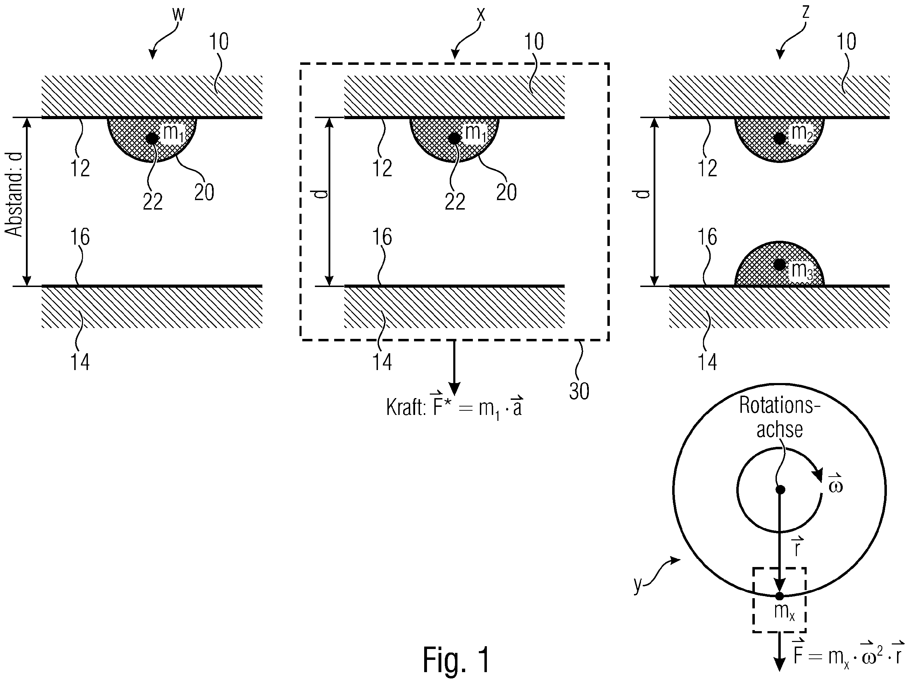

Ein Beispiel eines schematischen Ablaufs des Verfahrens und eines Aufbaus eines entsprechenden Systems sind in Fig. 1 dargestellt.

Fig. 1 zeigte eine Abgabeeinheit 10, beispielsweise in Form eines ersten Substrats, die eine Abgabeoberfläche 12 aufweist. Eine Empfangseinheit 14, beispielsweise in Form eines zweiten Substrats, weist eine Empfangsoberfläche 16 auf. Die Abgabeeinheit 10 und die Empfangseinheit 14 sind derart relativ zueinander angeordnet, dass sich die Abgabeoberfläche 12 und die Empfangsoberfläche 16 in radialer Richtung gegenüberliegen. Ein Abstand d zwischen den Oberflächen 12, 16 und ein Winkel zwischen denselben können im Wesentlichen beliebig eingestellt werden, solange die hierin beschriebenen Verfahren durchgeführt werden können. Bei Beispielen bleiben der Abstand d und der Winkel während der Durchführung des Verfahrens, d.h. während des Transfers eines oder mehrerer Partikel, konstant. Die Abgabeoberfläche 12 und die Empfangsoberfläche 16 sind derart zueinander ausgerichtet, dass bei einer Rotation derselben um eine Rotationsachse, der Teil eines Flüssigkeitstropfens, der sich von der Abgabeoberfläche 12 trennt, auf der Empfangsoberfläche 16 landet.

Wie in der Darstellung w in Fig. 1 zu sehen ist, befindet sich auf der Abgabeoberfläche 12 ein Flüssigkeitstropfen 20, in dem sich zumindest ein Partikel 22 befindet. Bei dem Tropfen 20 kann es sich beispielsweise um einen an der Abgabeoberfläche 12 hängenden Tropfen handeln. Der Flüssigkeitstropfen 20 mit dem Partikel 22 weist eine Masse $m_1$ auf. Das Volumen des Flüssigkeitstropfens, die Masse $m_1$ und der Kontaktwinkel zwischen der Flüssigkeit und der Abgabeoberfläche 12 sind dabei derart, dass ungeachtet der Erdgravitationskraft die Oberflächenkräfte, Oberflächenspannung und Oberflächenhaftung, den Flüssigkeitstropfen 20 stationär an einer Position auf der Abgabeoberfläche 12, die als Abgabeposition bezeichnet werden kann, hält.

Durch eine Drehung des Verbundsystems 30 aus Abgabeeinheit 10 und Empfangseinheit 14, das in der mittleren Darstellung in Fig. 1 durch gestrichelte Linien angedeutet ist, kann eine auf den Flüssigkeitstropfen 20 und den oder die darin befindlichen Partikel(n) 22 einwirkende Zentrifugalkraft $\vec{F}$ erzeugt werden, wie in der Darstellung x in Fig. 1 gezeigt ist. Die Kraft $\vec{F}$, welche

den Transfer ermöglicht, wird somit durch Zentrifugation des Verbundsystems (Substrat mit Partikel und Rezipient, zueinander ausgerichtet) erzeugt. Die anzuwendende Kraft wird durch eine gerichtete Beschleunigung $\vec{a}$ erzeugt, die durch zentrifugale Rotation des Systems bewirkt wird, d.h. $\vec{F} = m_1 \cdot \vec{a}$. Wie in der Darstellung y in Fig. 1 gezeigt ist, berechnet sich die Beschleunigung $\vec{a}$ bei einer Rotation um eine Rotationsachse mit der Rotationsgeschwindigkeit $\vec{\omega}$ bei einer Masse $m_x$ an einem Radius r wie folgt: $\vec{F} = m_1 \cdot \vec{\omega}^2 \cdot \vec{r}$. Die Beschleunigung hängt die Beschleunigung $\vec{a}$ somit von der Winkelgeschwindigkeit $\vec{\omega}^2$, sowie der Rotorlänge $\vec{r}$ der Zentrifuge ab. Die resultierende Kraft ist zusätzlich von der beschleunigten Masse $m_x$ abhängig.

Die Größe der erforderlichen Kraft ergibt sich aus den Benetzungseigenschaften des Systems aus Flüssigkeit und Oberfläche und kann bei Beispielen $\vec{a} = 10 \cdot g$ bis 500 · g (Erdbeschleunigung g = 9,81 m/s²) betragen.

Generell wirkt die Zentrifugalkraft in radialer Richtung bezüglich der Rotationsachse. Die Abgabeposition und die Empfangsposition sollten sich daher in radialer Richtung gegenüberliegen, so dass der Partikel in der Flüssigkeit durch die Zentrifugalkraft von der Abgabeposition zu der Empfangsposition transferiert werden kann. Bei Beispielen können die Abgabeeinheit und die Empfangseinheit derart relativ zu der Rotationsachse angeordnet sein, dass die Zentrifugalkraft senkrecht zu einer Abgabeoberfläche der Abgabeeinheit und einer Empfangsoberfläche der Empfangseinheit wirkt. Die Abgabeoberfläche und die Empfangsoberfläche können parallel zueinander sein.

Übersteigt die erzeugte Zentrifugalkraft die Kraft, die erforderlich ist, um die auf den Flüssigkeitstropfen 20 wirkenden Oberflächenkräfte zu überwinden, wird der Partikel 22 und ein Teil der Flüssigkeit des Flüssigkeitstropfens 20 mit der Masse $m_3$ von der Abgabeoberfläche 12 abgelöst und auf die gegenüberliegende Empfangsoberfläche 16 transferiert. Dies ist in der Darstellung z in Fig. 1 dargestellt. Auf der Abgabeoberfläche 12 verbleibt bei dem gezeigten Beispiel ein Teil der Flüssigkeit mit der Masse $m_2$. Die Krafterzeugung wird durch Rotation des Systems 30 beispielsweise in einer Zentrifuge bewirkt. Die Kraft $\vec{F}$ wird durch eine Beschleunigung $\vec{a}$ der zum Transfer vorgesehene Masse $m_x$, d. h. Tropfen 20 inkl. Partikel 22 erzeugt. Diese Beschleunigung ergibt sich wie oben beschrieben aus gegebener Winkelgeschwindigkeit $\vec{\omega}$ und definiertem Abstand $\vec{r}$ zur Rotationsachse innerhalb der Zentrifuge.

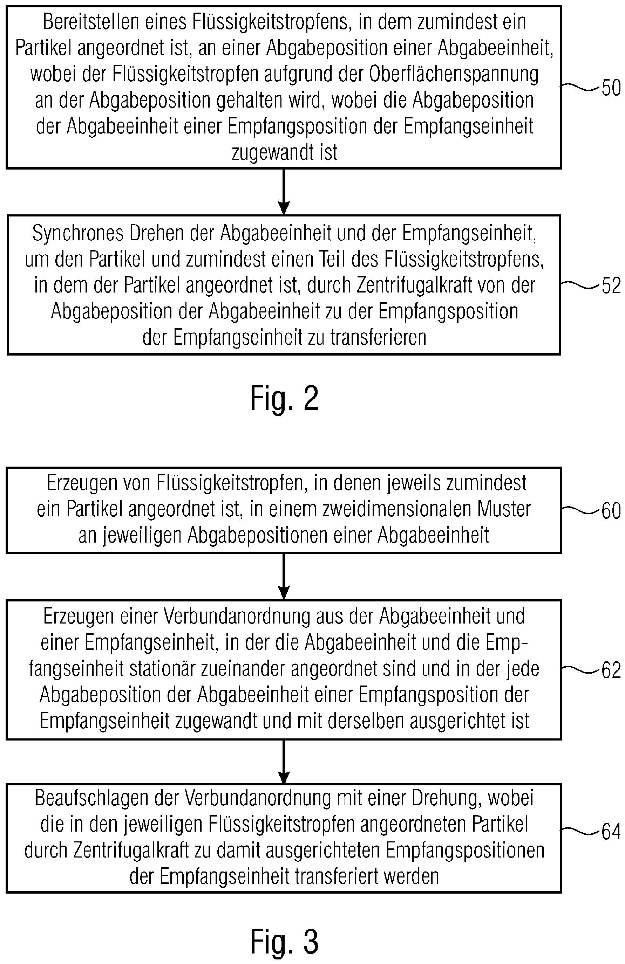

Beispiele der Erfindung schaffen somit ein Verfahren zum Transferieren zumindest eines Partikels von einer Abgabeeinheit 10 zu einer Empfangseinheit 14 wie es in Figur 2 gezeigt ist. Bei 50 wird ein Flüssigkeitstropfen 20, in dem zumindest ein Partikel 22 angeordnet ist, an einer Abgabeposition der Abgabeeinheit 10 bereitgestellt, wobei der Flüssigkeitstropfen 20 aufgrund der Oberflächenkräfte an der Abgabepositionen gehalten wird, wobei die Abgabeposition der Abgabeeinheit 10 einer Empfangsposition der Empfangseinheit 14 zugewandt ist, bzw. derselben in radialer Richtung gegenüberliegt. Bei 52 werden die Abgabeeinheit 10 und die Empfangseinheit 14 synchron gedreht, um den Partikel 22 und zumindest einen Teil des Flüssigkeitstropfens 20, in dem der Partikel 22 angeordnet ist, durch Zentrifugalkraft von der Abgabepositionen der Abgabeeinheit 10 zu der Empfangsposition der Empfangseinheit 14 zu transferieren.

Beispiele der Erfindung schaffen ein Verfahren, wie es in Figur 3 gezeigt ist. Bei 60 werden Flüssigkeitstropfen, in denen jeweils zumindest ein Partikel angeordnet ist, in einem zweidimensionalen Muster an jeweiligen Abgabepositionen einer Abgabeeinheit angeordnet. Bei 62 wird eine Verbundanordnung aus der Abgabeeinheit und einer Empfangseinheit erzeugt, in der die Abgabeeinheit und die Empfangseinheit stationär zueinander angeordnet sind und in der jede Abgabepositionen der Abgabeeinheit einer Empfangspositionen der Empfangseinheit zugewandt und mit derselben ausgerichtet ist. Bei 64 wird die Verbundanordnung mit einer Drehung beaufschlagt, wobei der oder die in den jeweiligen Flüssigkeitstropfen angeordneten Partikel durch Zentrifugalkraft zu den damit ausgerichteten Empfangspositionen der Empfangseinheit transferiert werden.

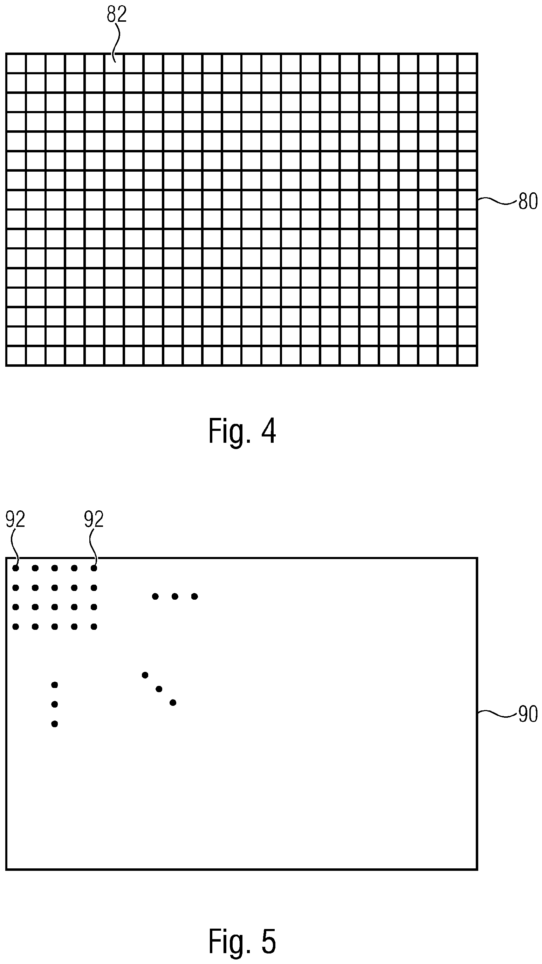

Beispiele ermöglichen somit einen parallelen Transfer mehrerer Partikel von einer Abgabeeinheit zu einer Empfangseinheit. Bei Beispielen ist die Empfangseinheit eine Mikrotiterplatte, wobei Fig. 4 schematisch eine Draufsicht auf eine Mikrotiterplatte 80 mit 364 Kavitäten 82, die in einem zweidimensionalen Muster oder Raster mit 16 Zeilen und 24 Spalten angeordnet sind. Figur 5 zeigt schematisch eine Draufsicht auf eine Abgabeeinheit in Form eines Deckels 90 einer Mikrotiterplatte. Der Deckel 90 ist ausgebildet, um auf der Mikrotiterplatte 80 derart angeordnet zu werden, dass die Kavitäten 82 der Mikrotiterplatte 80 geschlossen sind. Die Mikrotiterplatte 80 und der Deckel 90 bilden dann eine Verbundanordnung, die gemeinsam einer Rotation unterworfen werden kann.

Wie schematisch in Figur 5 angedeutet ist, werden auf einer Seite des Deckels 90 Flüssigkeitstropfen 92 erzeugt, in denen sich jeweils ein oder mehrere Partikel befinden. Beispielsweise können eine Anzahl von 364 Flüssigkeitstropfen auf dem Deckel 90 erzeugt werden, und zwar in einem Muster, dass der Anordnung der Kavitäten 82 in der Mikrotiterplatte 80 entspricht. Fig. 5 zeigt nur einige der erzeugten Flüssigkeitstropfen 92, während weitere lediglich durch jeweils drei Punkte in waagrechter Richtung, senkrechter Richtung und diagonale Richtung in Figur 5 angedeutet sind. Wenn der Deckel 90 auf der Mikrotiterplatte 80 angeordnet wird, ist jeweils einer der Flüssigkeitstropfen 92 einer Kavität 82 der Mikrotiterplatte 80 zugewandt und mit derselben ausgerichtet.

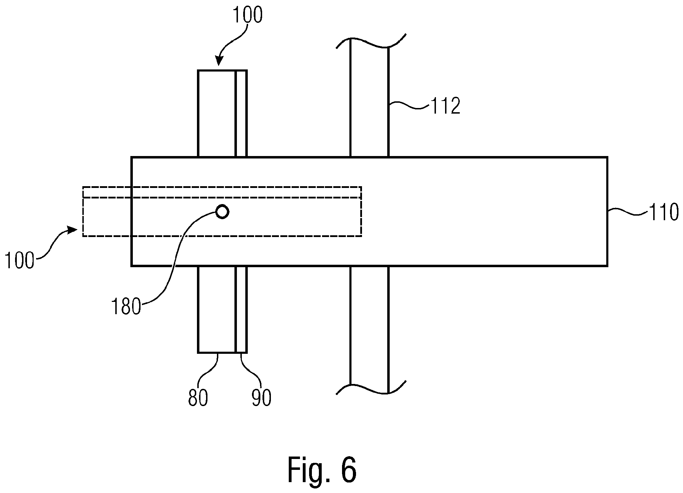

Die Verbundanordnung aus Mikrotiterplatte 80 und Deckel 90 kann dann in einer Zentrifuge platziert werden, wie dies schematisch in Fig. 6 gezeigt ist. Fig. 6 zeigt eine Verbundeinordnung 100 aus Mikrotiterplatte 80 und Deckel 90, die in einer Zentrifuge 110 angeordnet ist. Die Zentrifuge 110 ist um eine Achse 112 drehbar. Die Verbundanordnung 100 kann in der in Fig. 6 gezeigten Stellung in der Zentrifuge 110 angeordnet werden, so dass der Deckel 90 mit den auf denselben aufgebrachten Flüssigkeitstropfen bezüglich der Rotationsachse 112 radial inneren angeordnet ist. Durch Drehen der Zentrifuge können dann die Partikel, die sich in den Flüssigkeitstropfen 92 auf dem Deckel 90 befinden, durch Zentrifugalkraft von dem Deckel 90 in die Kavitäten 82 der Mikrotiterplatte 80 transferiert werden.

Bei alternativen Beispielen kann die Verbundanordnung 100 schwenkbar in der Zentrifuge 110 angeordnet sein. Dabei kann die Verbundanordnung 100 in einer ersten waagrechten Stellung, die in gestrichelten Linien in Fig. 6 angedeutet ist, in eine Schwenkvorrichtung der Zentrifuge eingebracht werden. Die Schwenkvorrichtung ist in Fig. 6 schematisch durch eine Schwenkachse 120 dargestellt. Die Schwenkvorrichtung ist derart ausgebildet, dass die Verbundanordnung 100 in die in Fig. 6 gezeigte vertikale Stellung, die eine Abgabestellung darstellt, schwenkt, wenn die Zentrifuge 110 einer Rotation unterworfen wird.

Bei Beispielen kann die Zentrifugation beispielsweise bei einer Umdrehung von 1000 bis 1500 Umdrehungen pro Minute, z.B. 1200 Umdrehungen pro Minute, für 5 Minuten durchgeführt werden. Bei Beispielen kann der Deckel mit den Flüssigkeitstropfen für eine bestimmte Inkubationszeit mit der Mikrotiterplatte verbunden bleiben, um eine Sphäroid-Bildung zu bewirken, bevor der Transfer der Flüssigkeitstropfen stattfindet. Beispielsweise kann ein Inkubationszeit 24 Stunden betragen, bei einer Temperatur von 37°C und 5% $CO_2$.

Bei Beispielen kann die Flüssigkeit des Flüssigkeitstropfens ein Sphäroid-Bildungsmedium mit einer definierten Konzentration von Zellen pro Volumen aufweisen. Beispielsweise kann die Konzentration $5 \cdot 10^5$ Zellen/ml betragen. Somit können beispielsweise Flüssigkeitstropfen eines Volumens von 1 μl, die jeweils 500 Zellen aufweisen, auf der Abgabeeinheit erzeugt werden.

Bei Beispielen kann oder können die zu der Empfangseinheit, wie z.B. der Mikrotiterplatte, transferierten Partikel, wie z.B. zellbasierten Partikel, einer weiteren Behandlung oder weiteren Behandlungen unterzogen werden, beispielsweise Behandlungen mit unterschiedlichen Medikamenten. Dadurch ist es bei Beispielen möglich, ein vollständiges In-Vitro-Screening durchzuführen, indem unterschiedliche Konzentrationen von Medikamenten unterschiedlichen Proben zugeführt werden, und/oder indem Proben mit unterschiedlichen Konzentrationen mit Medikamenten behandelt werden.

Bei Beispielen der vorliegenden Erfindung werden die Abgabeeinheit und die Empfangseinheit derart relativ zu einer Rotationsachse angeordnet, dass eine Drehung um die Rotationsachse eine Zentrifugalkraft auf den oder die Flüssigkeitstropfen bewirkt, die von der jeweiligen Abgabeposition zu der jeweiligen Empfangsposition gerichtet ist. Da im zentrifugalen Feld die Zentrifugalkraft radial nach außen wirkt, ist bei Beispielen zumindest in einer Abgabestellung die jeweilige Empfangsposition bezüglich der Rotationsachse radial weiter außen angeordnet als die Abgabeposition. Bei Beispielen ist die Ausrichtung der Rotationsachse nicht von Bedeutung. Die Rotationsachse kann bezüglich des Erdgravitationsfelds vertikal, horizontal oder in einem beliebigen Winkel angeordnet sein.

Bei Beispielen weist die Flüssigkeit, in der die Partikel suspensiert sind, bei Raumtemperatur (20 °C) eine Oberflächenspannung von maximal 72,75 mN/m auf. Das Volumen des Flüssigkeitstropfens beträgt bei Beispielen mindestens 0,5 μl vorzugsweise mindestens 1 μl. Bei Beispielen beträgt das Volumen der Flüssigkeit maximal 100 μl.

Wie dieser Ausdruck hierin verwendet ist, sind Partikel definiert als unlösliche Komponenten, bestehend aus einem oder mehreren der Folgenden;

- soliden Materialien (z.B. Polymer-Beads),
- halbfesten Materialien (z.B. Hydrogele) oder
- zellbasierte Partikel aus biologischen Zellen.

Zellbasierte Partikel aus biologischen Zellen können aufweisen:

- Einzel-Zellen
- Zell-Suspension
- Zell-Aggregate
- Sphäroide
- Organoide
- Gewebe-Probe aus Biopsie (z.B. Patientenproben)
- Kombinationen der genannten Elemente

Bei Beispielen werden das Material der Abgabeposition bzw. der Abgabeeinheit und/der das Material der Empfangsposition bzw. der Empfangseinheit so gewählt, dass der Kontaktwinkel zwischen der Flüssigkeit des Flüssigkeitstropfens und der Abgabeposition bzw. Empfangsposition mindestens 45° und maximal 135° beträgt.

Bei Beispielen kann die Abgabeeinheit eine flache, unstrukturierte Oberfläche eines soliden oder semisoliden Materials aufweisen, auf der der oder die Flüssigkeitstropfen bereitgestellt werden. Bei Beispielen kann die Abgabeeinheit eine flache, strukturierte Oberfläche eines soliden oder semisoliden Materials aufweisen, auf der der oder die Flüssigkeitstropfen bereitgestellt werden. Unter einer flachen Oberfläche ist dabei eine Oberfläche zu verstehen, in der keine Fluidikstrukturen gebildet sind, durch die die Flüssigkeit durch die Abgabeeinheit zu der Oberfläche zugeführt wird, wie z. B. Kanäle und/oder Düsen. Bei Ausführungsbeispielen kann die Oberfläche strukturiert sein, um eine oder mehrere Ausnehmungen aufzuweisen, in der oder den der oder die Flüssigkeitstropfen bereitgestellt werden. Bei Beispielen kann die Abgabeeinheit eine Mikrotiterplatte sein.

Bei Beispielen kann die Empfangseinheit eine flache, unstrukturierte Oberfläche eines soliden oder semisoliden Materials aufweisen, auf die der oder die Flüssigkeitstropfen transferiert werden. Bei Beispielen kann die Empfangseinheit eine flache, strukturierte Oberfläche eines soliden oder semisoliden Materials aufweisen, auf die der oder die Flüssigkeitstropfen transferiert werden. Bei Ausführungsbeispielen kann die Oberfläche der Empfangseinheit strukturiert sein, um eine oder mehrere Ausnehmungen aufzuweisen, in die der oder die Flüssigkeitstropfen transferiert wird bzw. werden. Bei Beispielen kann die Empfangseinheit getrennten Reaktionskammern oder Kavitäten aufweisen. Bei Beispielen kann die Empfangseinheit eine Mikrotiterplatte sein.

Bei Beispielen können die Abgabeeinheit und/oder die Empfangseinheit Kombinationen der beschriebenen Merkmale aufweisen.

Beispiele der Erfindung schaffen Verfahren zum Übertragen von Partikeln, die in mindestens einem auf einer ersten Oberfläche (Abgabeeinheit) anhaftenden Flüssigkeitstropfen suspensiert sind, auf eine zweite Oberfläche (Empfangseinheit) zur weiteren Analyse oder Verwendung, das ein Positionieren der ersten Oberfläche mit dem anhaftenden Tropfen gegenüber der zweiten Oberfläche, so dass die erste Oberfläche inklusive des/der Tropfen zum Rezipienten hin exponiert ist, und ein Beaufschlagen der zueinander ausgerichteten Oberflächen mit einer Zentrifugalkraft, die vom Tropfen zur Empfangseinheit gerichtet ist, z.B. durch Verwendung einer Zentrifuge, aufweist.

Bei Beispielen der Erfindung ist oder sind in dem oder jedem Flüssigkeitstropfen ein oder mehrere Partikel suspensiert. Bei Beispielen weisen der oder die Partikel Sphäroide bzw. Zellaggregate auf. Beispiele sind auf Verfahren zum Übertragen von zellbasierten Partikeln gerichtet, wobei unter solche zellbasierten Partikel Einzel-Zellen, Zell-Aggregate, d.h. mehr als eine Zelle in räumlicher Verbindung zueinander, Sphäroide, und Organoide fallen. Bei Beispielen sind der oder die Partikel in einem hängenden Tropfen (hanging drop), der an einer ersten Oberfläche der Abgabeeinheit anhaftet, suspensiert und werden auf eine zweite Oberfläche einer Empfangseinheit (Rezipient) zur weiteren Analyse oder Verwendung transferiert.

Bei Beispielen ist das Volumen des oder der an der ersten Oberfläche anhaftenden Flüssigkeitstropfen 1 µl oder mehr. Bei Beispielen sind das Volumen der Flüssigkeitstropfen und der Kontaktwinkel zwischen Flüssigkeitstropfen und Abgabeposition, d.h. Oberfläche der Abgabeeinheit, derart, dass der oder die Flüssigkeitstropfen allein aufgrund der Oberflächenkräfte an der Abgabeposition gehalten werden, selbst wenn der Tropfen im Erdgravitationsfeld nach unten an der Abgabeposition hängt. Bei Beispielen ist eine definierte Anzahl von Tropfen pro Tropfen in dem oder den Tropfen suspensiert. Bei Beispielen ist genau ein Partikel pro Tropfen in dem oder den Tropfen suspensiert.

Beliebige bekannte Mechanismen können bei Beispielen verwendet werden, um den oder die Flüssigkeitstropfen auf der Abgabeeinheit bereitzustellen. Beispielsweise können Dispensiervorrichtungen, die nach dem Drop-on-Demand-Prinzip arbeiten, verwendet werden. Beispiele solcher Dispensiervorrichtungen sind piezoelektrisch oder thermisch betriebene Drop-on-Demand-Dispenser. Alternativ können auch andere Dispensiervorrichtungen, wie z.B. solche, die ein Solenoid-Mikroventil aufweisen, verwendet werden. Andere Beispiele können einen Laser-induzierten Vorwärtstransfer oder ein akustisches Dispensieren verwenden, um den oder die Flüssigkeitstropfen bereitzustellen. Die Dispensiervorrichtung kann ausgebildet sein, um jeweils einen Flüssigkeitstropfen zu dispensieren, in dem eine definierte Anzahl von Zellen, wie z.B. exakt eine Zelle, suspensiert ist. Um dies sicherzustellen, können solche Dispenser mit optischen Überwachungseinrichtungen versehen sein, die ausgebildet sind, um sicherzustellen, dass nur solche Flüssigkeitstropfen, in denen die gewünschte Anzahl von Zellen enthalten ist, auf die Abgabeeinheit dispensiert werden.

Beispiele der hierin beschriebenen Erfindung ermöglichen eine hohe Parallelität des Transfers von Partikeln bzw. Proben von einer Abgabeeinheit zu einer Empfangseinheit. Bei Beispielen können Arrays mit vielen Tropfen verwendet werden. Beispiele ermöglichen eine variable Anordnung der Tropfen auf der Abgabeeinheit, beispielsweise dem Substrat, da die Abgabeeinheit eine flache Oberfläche aufweisen kann, die keine vordefinierte Merkmale, wie z.B. Reservoire/Kanäle/Düsen, aufweist. Beispiele ermöglichen geringe Kosten für benötigte Laborgeräte, da ausschließliche standardisierte Verbrauchsmaterialien und Geräte verwendet werden können. Beispiele ermöglichen eine sanfte Handhabung von Partikeln/Sphäroiden, da der Kontakt zu festen Teilen minimiert werden kann, indem der Transfer direkt von der Abgabeeinheit zu der Empfangseinheit unter Ausnutzung der Zentrifugalkraft erfolgt. Beispiele ermöglichen eine geringe Scherrate beim Transfer durch eine niedrige Weber- und Reynoldszahl beim Transfer. Beispiele ermöglichen ferner eine Wiederholbarkeit des Transfers, indem beispielsweise ein weiterer Transfer zurück auf die Abgabeeinheit oder ein weiterer Transfer auf eine weitere Empfangseinheit stattfindet. Bei einem solchen weiteren Transfer hat die Empfangseinheit des ersten Transfers dann jeweils die Funktion der Abgabeeinheit für den weiteren Transfer.

Verglichen mit bekannten Prozessen ermöglichen Beispiele der Erfindung, dass beliebige Partikel oder eine Mehrzahl von Partikeln transferiert werden können. Da der oder die Partikel in dem Flüssigkeitstropfen suspensiert sind und mit zumindest einem Teil der Flüssigkeit übertragen werden, haften diese selbst nicht an Oberflächen der Abgabeeinheit und/oder Empfangseinheit an, so dass kein Ablösen der Partikel selbst von solchen Oberflächen erforderlich ist. Beispiele der Erfindung können jegliche standardisierte und/oder unstrukturierte Oberflächen verwenden. Bei Beispielen kann die Anordnung der Partikel/Tropfen kann Nutzer-spezifisch mit beliebigem Rastermaß erfolgen. Bei Beispielen kann der Tropfentransfer parallelisiert und automatisiert erfolgen und kann beliebig oft wiederholt werden.

Beispiele der vorliegenden Erfindung beziehen sich auf Systeme, die konfiguriert sind, um die hierin beschriebenen Verfahren durchzuführen. Solche Systeme können eine oder mehrere der oben beschriebenen Komponenten aufweisen, die jeweils ausgelegt sind, um die beschriebenen Funktionalitäten zu implementieren. Obwohl einige Aspekte als Merkmale im Zusammenhang mit einem Verfahren beschrieben wurden, ist klar, dass eine solche Beschreibung auch als eine Beschreibung entsprechender Merkmale einer Vorrichtung/eines Systems bzw. der Funktionalität einer Vorrichtung/eines Systems betrachtet werden kann. Ferner klar, dass, wenn einige Aspekte der vorliegenden Offenbarung als Merkmale im Zusammenhang mit einer Vorrichtung beschrieben wurden, eine solche Beschreibung ebenfalls als eine Beschreibung entsprechender Verfahrensmerkmale betrachtet werden kann.

Die vorhergehende Offenbarung stellt Veranschaulichungen und Beschreibungen bereit, es ist jedoch nicht beabsichtigt, dass dieselbe erschöpfend ist oder die Implementierungen auf die offenbarte präzise Form eingeschränkt sind. Modifikationen und Variationen sind im Hinblick auf die obige Offenbarung möglich oder können aus der Praxis der Implementierungen erhalten werden. Obwohl bestimmte Kombination von Merkmalen in den Patentansprüchen angeführt und/oder in der Beschreibung offenbart sind, ist es nicht beabsichtigt, dass diese Merkmale die Offenbarung möglicher Implementierungen einschränken. Tatsächlich können zahlreiche dieser Merkmale auf Weisen kombiniert werden, die nicht spezifisch in den Patentansprüchen angeführt und/oder in der Beschreibung offenbart sind. Obwohl jeder der unten angeführten abhängigen Patentansprüche möglicherweise nur von einem oder einigen Patentansprüchen direkt abhängt, umfasst die Offenbarung möglicher Implementierungen jeden abhängigen Patentanspruch in Kombination mit allen anderen Patentansprüchen in dem Satz von Patentansprüchen.

Die oben beschriebenen Beispiele sind nur darstellend für die Grundsätze der vorliegenden Offenbarung. Es ist zu verstehen, dass Modifikationen und Variationen der Anordnungen und der Einzelheiten, die beschrieben sind, für Fachleute offensichtlich sind. Es ist daher beabsichtigt, dass die Offenbarung nur durch die beigefügten Patentansprüche und nicht durch die spezifischen Einzelheiten, die zum Zweck der Beschreibung und Erklärung der Beispiele dargelegt sind, begrenzt ist.

## Patentansprüche

1. Verfahren zum Transferieren zumindest eines Partikels in einer Flüssigkeit von einer Abgabeeinheit zu einer Empfangseinheit, mit folgenden Merkmalen:

   Bereitstellen eines Flüssigkeitstropfens, in dem der zumindest eine Partikel angeordnet ist, an einer Abgabeposition der Abgabeeinheit, wobei der Flüssigkeitstropfen aufgrund von Oberflächenkräften an der Abgabeposition gehalten wird, wobei die Abgabeposition der Abgabeeinheit einer Empfangsposition der Empfangseinheit zugewandt ist; und
   synchrones Drehen der Abgabeeinheit und der Empfangseinheit, um den Partikel und zumindest einen Teil des Flüssigkeitstropfens, in dem Partikel angeordnet ist, durch Zentrifugalkraft von der Abgabeposition der Abgabeeinheit zu der Empfangsposition der Empfangseinheit zu transferieren.

2. Verfahren nach Anspruch 1, bei dem die Abgabeposition der Abgabeeinheit eine flache Oberfläche der Abgabeeinheit ist.

3. Verfahren nach Anspruch 1 oder 2, bei dem die Abgabeeinheit eine flache Oberfläche aufweist, wobei das Bereitstellen des Flüssigkeitstropfens ein Bereitstellen einer Mehrzahl von Flüssigkeitstropfen, in denen jeweils zumindest ein Partikel angeordnet ist, in einem zweidimensionalen Muster an jeweiligen Abgabepositionen auf der flachen Oberfläche aufweist.

4. Verfahren nach Anspruch 3, bei dem beim Drehen die in den jeweiligen Flüssigkeitstropfen angeordneten Partikel in der Flüssigkeit zu damit ausgerichteten Empfangspositionen der Empfangseinheit transferiert werden.

5. Verfahren nach Anspruch 3 oder 4, bei dem das zweidimensionale Muster der Anordnung von Kavitäten in einer Mikrotiterplatte entspricht.

6. Verfahren nach Anspruch 5, bei dem die Empfangseinheit die Mikrotiterplatte ist, wobei die Abgabeeinheit und die Mikrotiterplatte vor dem Drehen derart zueinander angeordnet werden, dass jeweils mindestens einer der Flüssigkeitstropfen auf der Abgabeeinheit mit einer Kavität in der Mikrotiterplatte ausgerichtet ist.

7. Verfahren nach Anspruch 6, bei dem die Abgabeeinheit ein Deckel für die Mikrotiterplatte ist, wobei bei dem Anordnen des Deckels und der Mikrotiterplatte zueinander die jeweiligen Kavitäten der Mikrotiterplatte und die am Deckel anhaftenden Flüssigkeitstropfen angeordnet werden, so dass mindestens ein Flüssigkeitstropfen über jeweils einer der Kavitäten angeordnet ist.

8. Verfahren nach Anspruch 7, das ferner ein Einbringen der Mikrotiterplatte mit dem darauf angeordneten Deckel in eine Zentrifuge aufweist.

9. Verfahren nach Anspruch 8, bei dem die Mikrotiterplatte in einer waagrechten Stellung in eine Schwenkvorrichtung der Zentrifuge eingebracht wird, wobei aufgrund einer auf die Schwenkvorrichtung wirkende Zentrifugalkraft die Mikrotiterplatte in der Schwenkvorrichtung in eine Abgabestellung schwenkt, wenn die Zentrifuge rotiert, wobei in der Abgabestellung die Abgabepositionen radial innerhalb der Empfangspositionen sind und die Partikel durch Zentrifugalkraft von Abgabepositionen zu den Empfangspositionen transferiert werden.

10. Verfahren nach einem der Ansprüche 1 bis 9, bei dem der oder die Flüssigkeitstropfen, in denen jeweils zumindest ein Partikel angeordnet ist, unter Verwendung einer Dispensiervorrichtung auf die Abgabeeinheit aufgebracht wird oder werden.

11. Verfahren nach Anspruch 10, das ferner ein automatisches Ändern der Positionsbeziehung zwischen Dispensiervorrichtung und Abgabeeinheit aufweist, um eine Mehrzahl der Flüssigkeitstropfen, in denen jeweils zumindest ein Partikel angeordnet ist, in einem oder dem zweidimensionalen Muster auf der Abgabeeinheit zu erzeugen.

12. Verfahren nach einem der Ansprüche 1 bis 11, bei dem das Volumen des oder der Flüssigkeitstropfen in einem Bereich von 0,5 $\mu$l bis 100 $\mu$l liegt oder von 1 $\mu$l bis 100 $\mu$l liegt.

13. Verfahren nach einem der Ansprüche 1 bis 12, bei dem der oder die Partikel ein solides Material, ein halbfestes Material, zellbasierte Partikel aus biologischen Zellen, und/oder Kombinationen dieser Bestandteile aufweisen.

14. Verfahren nach einem der Ansprüche 1 bis 13, bei dem der Kontaktwinkel zwischen der Flüssigkeit des Flüssigkeitstropfens oder der Flüssigkeitstropfen und dem Material der Abgabeposition oder den Abgabepositionen der Abgabeeinheit 45° bis 135° beträgt.

15. Verfahren nach einem der Ansprüche 1 bis 14, bei dem während des Drehens ein Abstand und/oder ein Neigungswinkel zwischen der Abgabeposition und der Empfangsposition oder zwischen den Abgabepositionen und den Empfangspositionen konstant sind.

16. System, das ausgelegt ist, um ein Verfahren nach einem der Ansprüche 1 bis 15 durchzuführen.

Abstant: d

w

10

12

22    20

m₁

16

14

x

10

12

22    20

m₁

16

14

d

30

Kraft: $\vec{F}^* = m_1 \cdot \vec{a}$

z

10

12

m₂

16

m₃

14

d

Rotations-achse

$\vec{\omega}$

$\vec{r}$

y

m_x

$\vec{F} = m_x \cdot \vec{\omega}^2 \cdot \vec{r}$

Fig. 1

EP 3 815 788 A1

Bereitstellen eines Flüssigkeitstropfens, in dem zumindest ein Partikel angeordnet ist, an einer Abgabeposition einer Abgabeeinheit, wobei der Flüssigkeitstropfen aufgrund der Oberflächenspannung an der Abgabeposition gehalten wird, wobei die Abgabeposition der Abgabeeinheit einer Empfangsposition der Empfangseinheit zugewandt ist ⌇50

Synchrones Drehen der Abgabeeinheit und der Empfangseinheit, um den Partikel und zumindest einen Teil des Flüssigkeitstropfens, in dem der Partikel angeordnet ist, durch Zentrifugalkraft von der Abgabeposition der Abgabeeinheit zu der Empfangsposition der Empfangseinheit zu transferieren ⌇52

## Fig. 2

Erzeugen von Flüssigkeitstropfen, in denen jeweils zumindest ein Partikel angeordnet ist, in einem zweidimensionalen Muster an jeweiligen Abgabepositionen einer Abgabeeinheit ⌇60

Erzeugen einer Verbundanordnung aus der Abgabeeinheit und einer Empfangseinheit, in der die Abgabeeinheit und die Empfangseinheit stationär zueinander angeordnet sind und in der jede Abgabeposition der Abgabeeinheit einer Empfangsposition der Empfangseinheit zugewandt und mit derselben ausgerichtet ist ⌇62

Beaufschlagen der Verbundanordnung mit einer Drehung, wobei die in den jeweiligen Flüssigkeitstropfen angeordneten Partikel durch Zentrifugalkraft zu damit ausgerichteten Empfangspositionen der Empfangseinheit transferiert werden ⌇64

## Fig. 3

Fig. 4

Fig. 5

Fig. 6

Europäisches
Patentamt

European
Patent Office

Office européen
des brevets

# EUROPÄISCHER RECHERCHENBERICHT

Nummer der Anmeldung

EP 20 20 4424

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2003/032071 A1 (WANG EVELYN [US] ET AL) 13. Februar 2003 (2003-02-13) * Absatz [0059]; Abbildungen 14-18 * ----- | 1-16 | INV. B01L3/02 B01L3/00 C12M1/32 C12M1/12 |
| X | US 2014/106395 A1 (FATTINGER CHRISTOF [CH] ET AL) 17. April 2014 (2014-04-17) * Absätze [0077], [0078]; Ansprüche 27-29; Abbildungen 1-15 * ----- | 1-16 | |
| X | US 2015/196907 A1 (BEEBE DAVID J [US] ET AL) 16. Juli 2015 (2015-07-16) * Absätze [0082] - [0087], [0002]; Abbildungen 16-18 * ----- | 1-15 | |
| X | US 2019/293598 A1 (VANN CHARLES S [US] ET AL) 26. September 2019 (2019-09-26) * Absatz [0056]; Abbildung 4 * ----- | 1-16 | |
| X | US 2015/273469 A1 (REED MARK T [US] ET AL) 1. Oktober 2015 (2015-10-01) * Absätze [0398] - [0450]; Ansprüche 1-11 * * Absätze [0688] - [0691] * ----- | 1,3-16 | |
| X,D | DE 10 2007 018056 A1 (FRAUNHOFER GES FORSCHUNG [DE]; INST BIOPROZESS ANALYSENMESST [DE]) 23. Oktober 2008 (2008-10-23) | 16 | RECHERCHIERTE SACHGEBIETE (IPC) B01L C12M |
| A | * das ganze Dokument * ----- | 1-15 | |

Der vorliegende Recherchenbericht wurde für alle Patentansprüche erstellt

| Recherchenort | Abschlußdatum der Recherche | Prüfer |
|---|---|---|
| Den Haag | 15. März 2021 | Tiede, Ralph |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 20 20 4424

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-03-2021

| Im Recherchenbericht angeführtes Patentdokument | Datum der Veröffentlichung | Mitglied(er) der Patentfamilie | Datum der Veröffentlichung |
|---|---|---|---|
| US 2003032071 A1 | 13-02-2003 | KEINE | |
| US 2014106395 A1 | 17-04-2014 | BR 112013021837 A2 | 09-08-2016 |
| | | CA 2825615 A1 | 07-09-2012 |
| | | CN 103429729 A | 04-12-2013 |
| | | EP 2681305 A2 | 08-01-2014 |
| | | ES 2546159 T3 | 21-09-2015 |
| | | HK 1191970 A1 | 08-08-2014 |
| | | JP 5999518 B2 | 28-09-2016 |
| | | JP 2014506799 A | 20-03-2014 |
| | | KR 20140003562 A | 09-01-2014 |
| | | MX 341295 B | 12-08-2016 |
| | | RU 2013144195 A | 10-04-2015 |
| | | US 2014106395 A1 | 17-04-2014 |
| | | WO 2012117083 A2 | 07-09-2012 |
| US 2015196907 A1 | 16-07-2015 | US 2015196907 A1 | 16-07-2015 |
| | | US 2018318827 A1 | 08-11-2018 |
| US 2019293598 A1 | 26-09-2019 | AT 485888 T | 15-11-2010 |
| | | EP 1789195 A1 | 30-05-2007 |
| | | US 2006102477 A1 | 18-05-2006 |
| | | US 2010236930 A1 | 23-09-2010 |
| | | US 2012138462 A1 | 07-06-2012 |
| | | US 2014001202 A1 | 02-01-2014 |
| | | US 2014251811 A1 | 11-09-2014 |
| | | US 2014374259 A1 | 25-12-2014 |
| | | US 2015008129 A1 | 08-01-2015 |
| | | US 2015338372 A1 | 26-11-2015 |
| | | US 2015338373 A1 | 26-11-2015 |
| | | US 2018128778 A1 | 10-05-2018 |
| | | US 2019293598 A1 | 26-09-2019 |
| | | WO 2006026351 A1 | 09-03-2006 |
| US 2015273469 A1 | 01-10-2015 | KEINE | |
| DE 102007018056 A1 | 23-10-2008 | CL 2008000978 A1 | 02-01-2009 |
| | | DE 102007018056 A1 | 23-10-2008 |
| | | EP 2136921 A1 | 30-12-2009 |
| | | ES 2392975 T3 | 17-12-2012 |
| | | PL 2136921 T3 | 31-01-2013 |
| | | PT 2136921 E | 23-11-2012 |
| | | WO 2008125347 A1 | 23-10-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015158777 A1

- DE 102007018056 A1